(19) Europäisches Patentamt
European Patent Office
Office européen des brevets

(11) Publication number: 0 288 205 A2

(12) **EUROPEAN PATENT APPLICATION**

(21) Application number: 88303335.9

(22) Date of filing: 13.04.88

(51) Int. Cl.4: C07D 493/22 , A01N 43/90 , A61K 31/35 , //(C07D493/22, 313:00,311:00,311:00,307:00)

The title of the invention has been amended (Guidelines for Examination in the EPO, A-III, 7.3).

(30) Priority: 21.04.87 GB 8709327

(43) Date of publication of application: 26.10.88 Bulletin 88/43

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Baker, Geoffrey Harold Beecham Pharmaceuticals
Great Burgh Yew Tree Bottom Road
Epsom Surrey KT18 5XQ(GB)
Inventor: Dorgan, Roderick John Beecham Pharmaceuticals
Great Burgh Yew Tree Bottom Road
Epsom Surrey KT18 5XQ(GB)
Inventor: Banks, Rhona Mary Beecham Pharmaceuticals
Walton Oaks Dorking Road
Tadworth Surrey KT20 7NT(GB)
Inventor: Poulton, Mark Edward Beecham Pharmaceuticals
Walton Oaks Dorking Road
Tadworth Surrey KT20 7NT(GB)

(74) Representative: West, Vivien et al
Beecham Pharmaceuticals Great Burgh Yew Tree Bottom Road
Epsom Surrey KT18 5XQ(GB)

(54) **Macrolide derivatives with a parasiticidal activity, their preparation and compositions comprising them.**

(57) Compounds of formula (VIII):

EP 0 288 205 A2

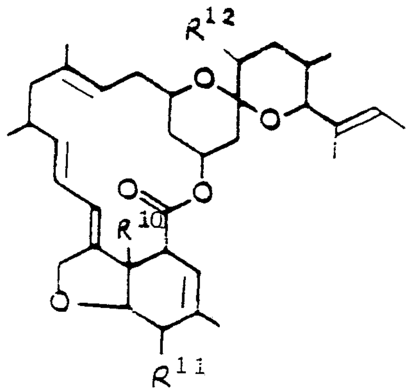

(VIII)

wherein $R^{10}$ is hydroxy or methoxy and $R^{11}$ and $R^{12}$ are the same or different and each is selected from optionally protected hydroxy, alkoxy, acyloxy, sulphonyloxy, oxo and optionally O-substituted oximino, but excluding VM44864 and VM44866 as herein defined, are new and useful as anthelmintic agents.

# NOVEL COMPOUNDS

The present invention relates to novel anthelmintically active materials, to processes for their production, to pharmaceutical formulations containing them, and to their use in human or veterinary medicine.

The milbemycins and avermectins are a group of macrolide antibiotics which have been prepared by the cultivation of microorganisms and are described in inter alia GB-A-1,390,336, J. Antibiotics 29(3), 76-14 to 76-16 and 29 (6), 76-35 to 76-42, GB-A-2 170 499, EP-A-0 073 660 and EP-A-0 204 421. They have anthelmintic activity.

EP-A-0 254 583 discloses a group of antiparasitic milbemycin compounds obtainable by the fermentation of a Streptomyces microorganism.

Two particular compounds disclosed in EP-A-0 254 583 are VM 44864 and VM 44866, which have been assigned the structures (I) and (II) respectively:

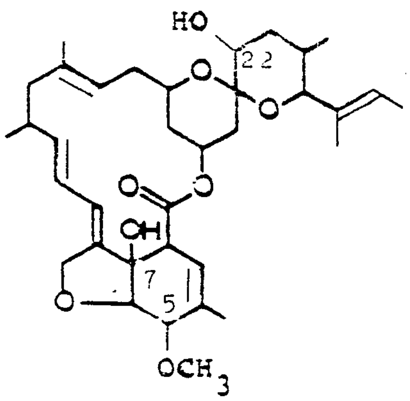

(I)

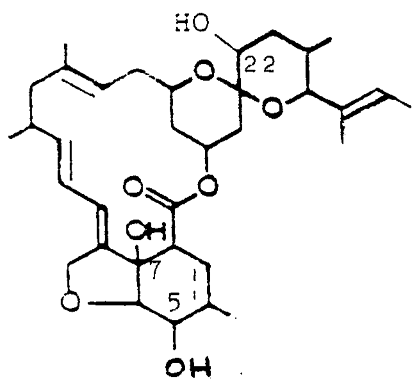

(II)

By analogy with known milbemycins, the absolute configuration of VM 44864 and VM 44866 is believed to be as follows:

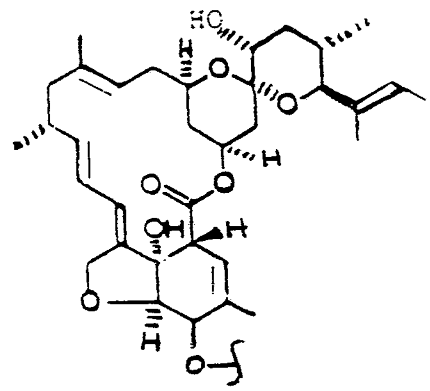

A first aspect of the invention provides a process for the preparation of VM 44864 which process comprises the methylation of VM 44866. The reaction is typically carried out using methyl iodide in the presence of a suitable silver salt or silver oxide.

A further aspect of the invention provides a process for the preparation of VM 44866 which process comprises reducing a ketone of formula (III):

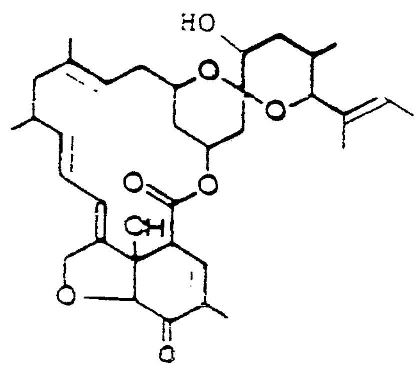

(III)

The reduction is typically carried out using sodium borohydride, which stereospecifically reduces the ketone (III) to VM 44866 having the configuration of the natural product.

The ketone (III) may be prepared by mercuric acetate oxidation of VM 44864 followed by acid hydrolysis, or by selective oxidation of VM 44866 with a suitable oxidizing agent such as manganese dioxide.

The ketone (III) is a novel compound and also forms a part of the present invention.

VM 44864 and VM 44866 have up to three hydroxyl groups which may be progressively alkylated using a suitable alkylating agent such as an alkyl iodide in the presence of a suitable silver salt or silver oxide, and such processes and the products obtainable thereby also form a part of the present invention.

A particular aspect of the invention thus provides compounds of formula (IV):

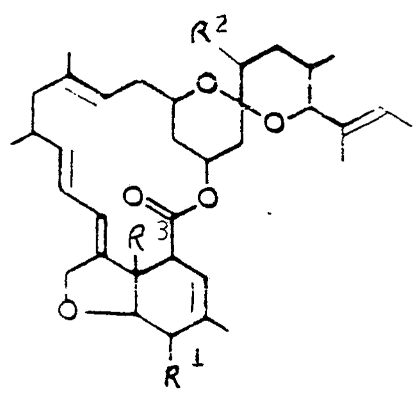

(IV)

wherein $R^1$ is alkoxy, $R^2$ is hydroxy or alkoxy, and $R^3$ is hydroxy or methoxy, with the proviso that when $R^3$ is methoxy $R^2$ is also methoxy, and when $R^1$ is methoxy $R^2$ is alkoxy.

As used herein alkoxy includes straight or branched $C_{1-6}$ alkoxy such as methoxy.

Selective alkylation of the $C_{22}$-OH of VM44866 may be achieved by protecting the $C_5$-OH prior to the alkylation. Suitable protecting groups are, for example, acyl or TBDMS (t-butyldimethylsilyl). Further suitable protecting groups are described in, for example, "Protective Groups in Organic Synthesis" Theodora W. Greene, Wiley-Interscience 1981 Ch 2, 10-86.

Acylation and sulphonylation of VM 44864 and VM 44866 may be effected using conventional techniques, for example by using the acid anhydride, or the acyl/sulphonyl halide in the presence of a base, for example pyridine or triethylamine, and such processes and the products obtainable thereby also form a part of the present invention.

A particular aspect of the invention thus provides compounds of formula (V):

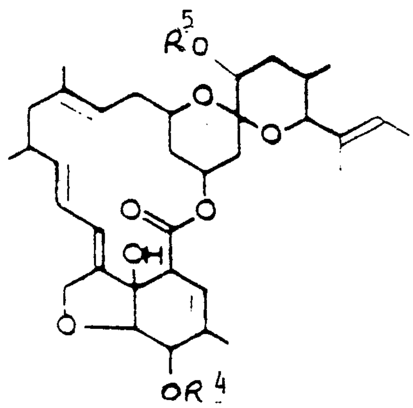

(V)

wherein $R^4$ is methyl, acyl or sulphonyl and $R^5$ is acyl or sulphonyl.

As used herein acyl includes $C_{1-6}$ alkanoyl, aryl-carbonyl, aryl($C_{1-6}$)alkanoyl, $C_{1-6}$alkoxycarbonyl, aryloxycarbonyl and aryl($C_{1-6}$)alkoxycarbonyl such as benzyloxycarbonyl, and acylation includes acylation with dicarboxylic acids to give the diester or the half ester.

Selective acylation/sulphonylation of the $C_5$-OH or $C_{22}$-OH of VM 44866 may be achieved by protecting the other of the two OH groups prior to the acylation/sulphonylation. Suitable protecting groups include the silyl derivatives such as TBDMS.

VM 44864 and VM 44866 may be oxidized to corresponding ketones using a suitable oxidizing agent such as manganese dioxide, which selectively oxidizes at the $C_5$ position, or DMSO/oxalyl chloride (Swern oxidation), which (additionally) oxidizes at the $C_{22}$ position, and such processes and the products obtainable thereby also form part of the present invention.

A particular aspect of the invention thus provides compounds of formula (VI):

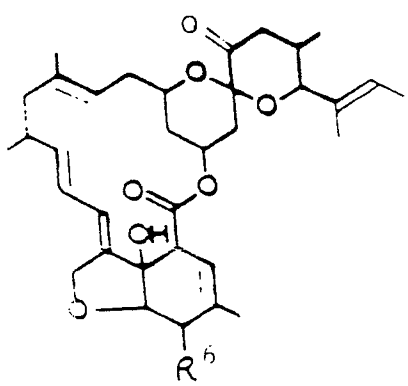

(VI)

wherein $R^6$ is methoxy or oxo.

Selective oxidation of the $C_{22}$-OH of VM 44866 may be achieved by protecting the $C_5$-OH prior to the oxidation. Suitable protecting groups include the silyl derivatives such as TBDMS, and acyl.

Reduction of a $C_5$ or $C_{22}$-keto group can be achieved using sodium borohydride as described above, to give essentially a product having the configuration of the natural product. Another reducing agent is lithium tri-sec-butyl borohydride, which has been found to give essentially a product having the opposite configuration to the natural product when used to reduce a $C_{22}$-keto group.

A broad aspect of the invention provides compounds of formula (VII):

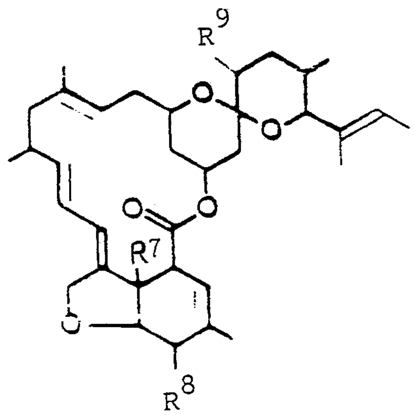

(VII)

wherein $R^7$ is hydroxy or methoxy, preferably hydroxy, and $R^8$ and $R^9$ are the same or different and each is selected from optionally protected hydroxy, alkoxy, acyloxy, sulphonyloxy and oxo, but excluding VM44864 and VM44866 as herein defined.

Compounds of formula (VII) may be prepared by a suitable combination of steps selected from alkylation, acylation, sulphonylation, oxidation, reduction, protection, and deprotection, as described hereinabove.

Compounds of the present invention having at least one oxo group may be converted to a corresponding optionally O-substituted oxime by conventional means, for example by reaction with optionally O-substituted hydroxylamine, for example in the form of the hydrochloride, in a suitable solvent such as aqueous methanol, typically at ambient temperatures. Once formed an O-substituted oxime may be reacted with a suitable O-acylating or O-alkylating agent. The compounds may exist as syn or anti isomers or mixtures thereof.

Suitable etherifying substituents for an oxime include $C_{1-6}$ alkyl and $C_{2-6}$ alkenyl.

Thus, a further broad aspect of the invention provides compounds of formula (VIII):

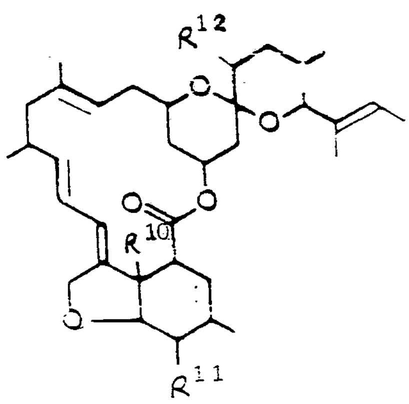

(VIII)

wherein $R^{10}$ is hydroxy or methoxy, preferaby hydroxy, and $R^{11}$ and $R^{12}$ are the same or different and each is selected from optionally protected hydroxy, alkoxy, acyloxy, sulphonyloxy, oxo and optionally O-substituted oximino, but excluding VM44864 and VM44866 as herein defined.

Each compound according to the invention is suitably provided in substantially pure form, for example at least 50% pure, suitably at least 60% pure, advantageously at least 75% pure, preferably at least 85%

pure, more preferably at least 95% pure, especially at least 98% pure, all percentages being calculated as weight/weight. An impure or less pure form of the compound according to the invention may, for example, be used in the preparation of a more pure form of each same compound or of a related compound (for example a corresponding derivative) suitable for pharmaceuticial use.

The compounds of the invention have parasiticidal properties, for example against nematodes such as Trichostrongulus colubriformis, and are useful for the treatment of helminthiasis in animals such as mammals, including humans and domesticated animals (including farm animals.)

Accordingly the present invention also provides a compound according to the invention, for use in the treatment of the human or animal body, especially for treating endo-and ectoparasitic infestations and particularly for treating helminthiasis of domestic and farm animals.

The term helminthiasis encompasses those diseases of man and animals caused by infestation with parasitic worms such as Strongyles, Ascarids, hookworms lungworms, filarial worms and whipworms. The compound may also be used against nematodes occurring in the soil or parasitic to plants.

The compounds of the invention are also active against Arthropods. The phylum Arthropoda comprises insects - such as biting flies, lice, bugs, beetles and fleas - and arachnids - such as mites and ticks.

Thus, a broad aspect of the invention provides a method of eradicating arthropod or nematode infestations, which method comprises applying a compound according to the invention or a derivative thereof to the arthropods or nematodes or to their environment.

The present invention thus provides a pesticidal composition comprising a compound according to the invention or a derivative thereof together with a suitable carrier or excipient, such as an aerosol formulation.

The present invention also provides a pharmaceutical or veterinary composition comprising a compound according to the invention or a pharmaceutically acceptable derivative thereof together with a pharmaceutically or veterinarily acceptable carrier or excipient.

The present invention also provides a method of treatment or prophylaxis of endo-and ectoparasitic infestations, especially helminthiasis, of animals, especially humans and domesticated mammals, which comprises administering an effective non-toxic amount of a compound according to the invention or a pharmaceutically acceptable derivative thereof, or a composition according to the invention, to a patient in need thereof.

The composition according to the invention may be formulated for administration in any convenient way for use in human or veterinary medicine, by analogy with other anthelmintics.

In suitable formulations the drug may be administered to animals orally (as a paste, drench, bolus, capsule or tablet), parenterally, percutaneously, as a food additive (eg granules, pellets or powder), or may be prepared as an aerosol spray formulation.

The compound of the invention may be formulated as a mixture with other anthelmintics, insecticides, acaricides or other pharmacologically active substances.

Suitably the composition consists of sufficient material to provide a dose of from 0.01 to 10mg of active ingredient per kg of animal body weight per dose, more suitably 0.1 to 1mg/kg per dose.

A composition according to the invention may suitably contain from 0.1% by weight, preferably from 10 to 60% by weight, of the compound according to the invention (based on the total weight of the composition), depending on the method of administration.

In certain cirumstances the crude fermentation broth may be administered, for example by incorporating the freeze-dried fermentation broth into the feed of the animal.

It will be appreciated that, in some cases, it will be advisable to repeat the dosing of the infected or potentially infected human or animal with the compound of the invention according to conventional dosage regimes used with anthelmintics.

The following Examples illustrate the invention.

Example 1

Methylation of VM 44866

A solution of VM 44866 (prepared as described in Example 3 of EP-A-0 254 583) (3 mg) in dry ether (3 ml) was stirred with methyl iodide (30 mg) and freshly prepared silver oxide (10 mg) for 15 h at room temperature. Examination of the reaction mixture by HPLC showed that all starting material had gone and the reaction mixture contained, as the major component, the 5-methoxy derivative VM 44864.

Example 2

5-Keto VM 44866

VM 44864 (prepared as described in Example 2 of EP-A-0 254 583) (1.76 g) and mercuric acetate (2.7 g) in toluene (30 ml) were heated to 100° under a nitrogen atmosphere and maintained at this temperature for 40 min. The mixture was cooled, ethyl acetate (250 ml) added and the solution washed sequentially with water, sodium bicarbonate solution and water. The ethyl acetate solution was dried ($MgSO_4$), filtered and evaporated to dryness. The residue was dissolved in glacial acetic acid (30 ml) and left at 20° overnight. The acetic acid was evaporated under high vacuum and the residue purified by column chromatography (silica eluted with hexane/ether mixtures 0 - 100% ether) to give the title compound (0.6 g). m/z (FAB $Na^+$ NOBA) (relative intensity) 619 [$MNa^+$] (100%)

$\delta_c$($CDCl_3$) 192.2, 172.0, 143.7, 138.5, 137.7, 137.2, 136.6, 134.1, 123.7, 123.4, 121.8, 120.7, 98.9, 81.9, 80.9, 71.6, 69.8, 69.3, 68.0, 48.4, 46.6, 36.8, 36.5, 36.3, 36.1, 34.6, 32.1, 22.2, 17.5, 15.6, 15.5, 13.2, 11.0, ppm

Example 3

Reduction of 5-Keto VM 44866 to give VM 44866

A solution of the ketone [crude material obtained from VM 44864 (34 mg) by the method of example 2] was dissolved in ethanol (10 ml), cooled to 0° and sodium borohydride (60 mg) added. After 1 h excess sodium borohydride was decomposed with dilute acetic acid solution, chloroform (100 ml) was added and the solution washed with water, dried and evaporated. The residue was purified by preparative TLC (silica/ether) to yield VM 44866 (19 mg) as a white foam. The product was identical (TLC, HPLC, NMR and mass spectra) to the natural product.

Example 4

5-Keto VM 44866

VM 44866 (3 mg) in dry ether (10 ml) and manganese dioxide (10 mg) were stirred at 20° for 2 h to give the title compound, identical by TLC and HPLC with an authentic sample.

Example 5

22-Methoxy VM 44864 and 7,22-Dimethoxy VM 44864

To a stirred suspension of silver oxide (20 mg) in dry ether (4 ml) containing VM 44864 (6.6 mg) was added methyl iodide (0.1 ml). The mixture was stirred at room temperature, in the dark, for 20 h. Silver oxide (10 mg) and methyl iodide (0.05 ml) were added and stirring continued for a further 24 h. The mixture was filtered, and the filtrate evaporated to an oil which was purified by preparative thin layer chromatography (silica plates eluted with methanol/dichloromethane 3:97) to give the title compounds.

22-Methoxy VM 44864 (2.1 mg); $\lambda_{max}$ ($CH_3OH$) 244 nm, m/z (FAB $Na^+$/Noba) (relative intensity) 635

(100%) $[MNa]^+$.

7,22-Dimethoxy VM 44864 (1.2 mg); $\lambda_{max}$ ($CH_3OH$) 245 nm, m/z (FAB $Na^+$ Noba) (relative intensity) 649 (100%) $[MNa]^+$.

Example 6

22-Methanesulphonyl VM 44864

A solution of VM 44864 (18 mg) in dry pyridine (3 ml) was treated with methanesulphonyl chloride (0.1 ml) and the mixture was stirred at room temperature for 5 minutes. The mixture was evaporated to dryness and dichloromethane (5 ml) was added. After washing the dichloromethane solution with water (2 $\times$ 5 ml) the organic layer was dried ($MgSO_4$), filtered and evaporated to give an oil. Purification by preparative thin layer chromatography (silica plates eluted with 2% methanol in dichloromethane) gave the title compound (12.6 mg), $\lambda_{max}$ ($CH_3OH$) 244 nm, m/z (FAB $Na^+$ Noba) 699 $[MNa]^+$.

Example 7

22-Acetoxy VM 44864

To a solution of VM 44864 (6.0 mg) in dry pyridine (1 ml) was added acetic anhydride (0.15 ml) and the mixture was stirred at room temperature for 24 hours. The mixture was evaporated to dryness and dichloromethane (5 ml) was added. After washing the dichloromethane solution with sodium bicarbonate solution (5 ml) the organic layer was dried ($MgSO_4$), filtered and evaporated to give an oil (8 mg). The product was purified by preparative thin layer chromatography (Analtech taper plate, silica, 2:98 methanol:dichloromethane) to give the title compound (2.1 mg), $\lambda_{max}$ ($CH_3OH$) 244 nm, m/z (FAB $Na^+$/Noba) (relative intensity) 663 $[MNa]^+$ (100%).

$\delta_c$($CDCl_3$) 173.7, 170.7, 142.5, 139.9, 137.2, 135.9, 133.9, 123.9, 123.4, 120.7, 119.5, 118.3, 97.7, 81.8, 80.3, 77.5, 76.9, 73.3, 68.3, 68.2, 67.9, 57.7, 48.5, 45.6, 36.6, 36.1, 35.9, 34.6, 32.2, 22.3, 21.3, 19.9, 17.3, 15.6, 13.1, 10.9 ppm.

Example 8

22-Keto VM 44864

To a solution of oxalyl chloride (0.05 ml) in dry dichloromethane (1 ml) at -70°, under a nitrogen atmosphere, was added dropwise a solution of dimethylsulphoxide (0.05 ml) in dry dichloromethane (1 ml). To this solution was added dropwise a solution of VM 44864 (8 mg) in dry dichloromethane (0.5 ml) and the resulting solution stirred at -70° for 1.5 h. A solution of triethylamine (0.2 ml) in dry dichloromethane (1 ml) was added, and the mixture allowed to warm to room temperature whilst stirring for 1.25 h. The mixture was poured onto a 1:1 mixture of cold water and ether. The layers were separated, the aqueous layer extracted with ether and the combined ether extracts washed with water, brine, and dried ($MgSO_4$). The ether was evaporated and the product purified by preparative thin layer chromatogoraphy (silica plate eluted with methanol/dichloromethane 2:98) to give the title compound (1.4 mg), $\lambda_{max}$ ($CH_3OH$) 244 nm, m/z (FAB $Na^+$ Noba) (relative intensity) 619 $[MNa]^+$ (100%).

$\delta_c$($CDCl_3$) 202.5, 173.9, 142.4, 140.0, 137.7, 136.0, 133.2, 124.9, 123.7, 120.2, 119.5, 118.4, 99.2, 81.8, 80.4, 77.6, 77.0, 69.1, 68.4, 68.3, 57.8, 48.6, 45.7, 43.3, 37.1, 36.3, 36.0, 34.6, 33.9, 22.4, 20.0, 18.2, 15.6, 13.3, 10.9 ppm.

Example 9

5-TBDMS-protected VM 44866 and 5,22-diTBDMS-protected VM44866

A mixture of VM 44866 (10.9 mg), imidazole (20 mg), t-butyldimethylsilylchloride (30 mg) and anhydrous dimethylformamide (5 ml) was stirred at 20°C for 16 h in an atmosphere of nitrogen. To the mixture was added t-butyldimethylsilylchloride (30 mg), imidazole (20 mg) and stirring continued at 21°C for 5 h. The mixture was poured onto water (10 ml) and extracted with ether (2 × 5 ml). The combined ether extracts were washed with water (10 ml), dried ($MgSO_4$) and evaporated to give an oil. This residue was purified by preparative thin layer chromatography (silica plate eluted with ether hexane 30/70) to give two products.

Product.1. 5-t-butyldimethylsilyl VM44866 (6.0 mg); t.l.c. $R_f$ = 0.33 ether hexane 1:1 $SiO_2$; m/z (FAB $Na^+$/Noba) (relative intensity) 721 $[MNa]^+$ (100%).

Product.2. 5,22-di-t-butyldimethylsilyl VM44866 (1.0 mg); t.l.c. $R_f$ = 0.66 ether/hexane 1:1 $SiO_2$; m/z (FAB $Na^+$ Noba) (relative intensity) 835 $[MNa]^+$ (100%).

Example 10

5-TBDMS-protected 22-keto VM 44866

A solution of oxalyl chloride (0.12 ml) in dry dichloromethane (2 ml) at -70°C under nitrogen was treated dropwise with a solution of dimethylsulphoxide (0.1 ml) in dry dichloromethane (2 ml) and then dropwise with a solution of 5-t-butyldimethylsilyl VM 44866 (46 mg) in dry dichloromethane (1.0 ml). The resulting solution was stirred at -70° for 1.5 h before being treated dropwise with a solution of triethylamine (0.4 ml) in dry dichloromethane (1 ml). The reaction mixture was stirred for 1 h without cooling and poured onto a mixture of cold water and ether (1:1). The aqueous layer was extracted with ether. The combined organic layers were washed with water, dried ($MgSO_4$) and evaporated. The residue was chromatographed over silica using dichloromethane increasing to dichloromethane/methanol 98:2 to give the title compound (16 mg).

Example 11

22-Keto VM 44866

A solution of 5-t-butyldimethylsilyl-22-keto VM 44866 (16 mg) in methanol (5 ml) was treated with p-toluenesulphonic acid (10 mg) and the mixture stirred for 1.5 h at 21°C. The mixture was then evaporated to dryness before addition of water (10 ml) and ether (10 ml). After separation. the ether layer was washed with water (10 ml), dried ($MgSO_4$) and evaporated to give an oil. This residue was purified by preparative thin layer chromatography (silica: dichloromethane/methanol 98:2) to give the title compound (10 mg) m/z (FAB $Na^+$ Noba) (relative intensity) 605 $[MNa]^+$ (100%).

$\delta_c$ ($CDCl_3$), 202.5, 173.7, 142.7, 139.7, 137.9, 137.7, 133.2, 124.9, 123.5, 120.3, 120.2, 118.0, 99.2, 81.8, 80.2, 79.2, 69.1, 68.5, 68.4, 67.8, 48.5, 45.7, 43.3, 37.1, 36.3, 36.0, 34.6, 33.9, 22.4, 19.9, 18.2, 15.6, 13.3, 10.9, ppm.

Example 12

22-hydroxyimino VM44864

To a solution of 22-keto VM 44864 (18mg) in methanol (5ml) was added dropwise a solution of hydroxylammonium chloride (24mg) in water (1ml). The resulting solution was stirred at room temperature for 15 minutes before evaporation at reduced pressure to remove the methanol. Water (15ml) and ether (15ml) were added and the ether layer was separated, washed with water (15ml), dried ($MgSO_4$), and evaporated. The crude product was purified by preparative thin layer chromatography (silica eluted with 5/95 $MeOH/CH_2Cl_2$) to give the title compound (10mg) m/z (FAB $Na^+$/Noba) (relative intensity)634 $[MNa]^+$ 100%

$\delta_c$ ($CDCl_3$), 173.8, 156.8, 142.4, 139.8, 137.3, 135.9, 133.7, 124.1, 123.5, 120.5, 119.5, 118.5, 98.0, 81.9, 80.4, 77.6, 76.9, 68.7, 68.4, 68.3, 57.8, 48.5, 45.7, 36.4, 35.9, 35.8, 34.5, 32.9, 27.1, 22.4,19.9, 17.7, 15.6, 13.2, 10.9 ppm.

Example 13

5-Hydroxyimino VM 44866

To a solution of 5-keto VM 44866 (30 mg) in methanol (2 ml) was added a solution of hydroxylamine hydrochloride (10 mg) in water (0.5 ml). The mixture was stirred at 20° for 6 h, evaporated, and dichloromethane added. The suspension was washed with water and the organic phase dried ($MgSO_4$) and evaporated. The crude product was purified by chromatography (silica eluted with ether/petrol 80:20) to give the title compound (10 mg). $\delta_C$ ($CDCl_3$) 173.1, 151.5, 142.9, 138.0, 137.2, 134.1, 131.9, 125.5, 123.7, 123.6, 121.3, 120.6, 98.9, 81.9, 78.5, 73.0, 71.6, 68.8, 68.7, 68.1, 48.5, 46.4, 36.9, 36.5, 36.3, 36.1, 34.6, 32.1, 22.2, 17.5, 17.5, 15.6, 13.1, 11.0 ppm.

Example 14

22-Methoxyimino VM 44864

A solution of 22-keto VM 44864 (12 mg) was treated with methoxylamine hydrochloride (25 mg) in an analogous procedure to that described in Example 12 to give the title compound (6.0 mg), m/z (FAB $Na^+$/Noba) (relative intensity) 648, $[MNa]^+$ (100%).

Example 15

22-Allyloxyimino VM 44864

A solution of 22-keto VM 44864 (30 mg) was treated with o-allylhydroxylamine hydrochloride (20 mg) in an analogous procedure to that described in Example 12 to give the title compound (14 mg), m/z (FAB $Na^+$/Noba) (relative intensity 674, $[MNa]^+$ (100%).

Example 16

22-Epi-VM 44864

To a solution of 22-keto VM 44864 (37 mg) in anhydrous THF (5 ml) at -78°C under a nitrogen atmosphere was added lithium tri-sec-butyl-borohydride, 1.0M solution in THF (0.2 ml). After stirring at -78°C (5 min) the mixture was allowed to warm to 0°C (45 min) before careful addition of water (5 ml) and ether (5 ml). After separation the ether layer was washed with water (5 ml), dried ($MgSO_4$) and evaporated to give an oil. This residue was purified by preparative thin layer chromatography (silica plate eluted with methanol: dichloromethane 3:97) to give the title compound (20 mg), m/z (FAB $Na^+$ Noba) (relative intensity) 621, $[MNa]^+$ (100%). (Found $M^+$, 598.3486, $C_{35}H_{50}O_8$ requires 598.3506), $\delta_C$ ($CDCl_3$) 173.8, 142.4, 139.8, 137.2, 136.0, 134.2, 123.6, 123.5, 120.6, 119.5, 118.4, 99.1, 82.2, 80.3, 77.5, 76.9, 70.0, 68.7, 68.3, 68.1, 57.7, 48.5, 45.7, 37.3, 36.3, 35.9, 35.1, 34.6, 25.3, 22.3, 19.9, 17.4, 15.5, 13.1, 11.1 ppm.

Example 17

22-Epi-acetoxy VM 44864

To a solution of 22-epi-VM 44864 (34 mg) in dry pyridine (5 ml) was added acetic anhydride (0.4 ml) and the mixture stirred for 4 days at room temperature. The product was isolated and purified in a similar manner to that described in Example 7, to give the title compound (31 mg), m/z (EI). (Found $M^+$ 640.3601, $C_{37}H_{52}O_9$ requires 640.3611), $\delta_C$ ($CDCl_3$) 173.8, 170.3, 142.4, 139.7, 137.2, 135.9, 134.4, 123.5, 123.5, 120.6, 119.4, 118.4, 97.6, 82.3, 80.3, 77.5, 77.0, 71.2, 68.5, 68.2, 67.9, 57.7, 48.5, 45.6, 36.9, 36.3, 35.9, 34.5, 32.6, 25.8, 22.3, 21.2, 19.9, 17.2, 15.5, 13.1, 11.0 ppm.

Example 18

22-Epi-VM 44866

A solution of 5-TBDMS-22-keto VM 44866 (34 mg) in THF was reduced with lithium tri-sec-butylborohydride in a procedure analogous to that of Example 16. The product was treated with p-toluenesulphonic acid to remove the TBDMS protecting group as described in Example 11 to give, after chromatography, the title compound (10 mg), m/z (FAB $Na^+$ Noba) (relative intensity) 607 $[MNa]^+$ (100%), $\delta_C$ ($CDCl_3$) 173.7, 142.7, 139.5, 137.8, 137.2, 134.1, 123.7, 120.6, 120.2, 118.0, 99.1, 82.3, 80.2, 79.1, 70.1, 68.7, 68.4, 68.1, 67.7, 48.5, 45.7, 37.3, 36.3, 36.0, 35.1, 34.6, 25.3, 22.3, 19.9, 17.4, 15.5, 13.1, 11.1 ppm.

Example 19

22-Hydroxyimino VM 44866

A solution of 22-keto VM 44866 (30 mg) in methanol was treated with hydroxylamine hydrochloride (50 mg) in a procedure analogous to that described in Example 12 to give, after chromatography, the title compound (12 mg), m/z (FAB $Na^+$ Noba) (relative intensity) 670, $[MNa]^+$ (100%), $\delta_C$ ($CDCl_3$) 173.7, 156.9, 142.7, 139.5, 137.8, 137.3, 133.7, 124.2, 123.4, 120.5, 120.2, 118.1, 97.9, 81.9, 80.2, 79.2, 68.6, 68.5, 68.4, 67.7, 48.4. 45.7, 36.4, 35.9, 35.8, 34.5, 32.9, 27.1, 22.3, 19.9, 17.7, 15.5, 13.2, 10.8 ppm.

Temperatures given in the Examples are in Celsius unless otherwise indicated.

## Claims

1. A compound of formula (VIII):

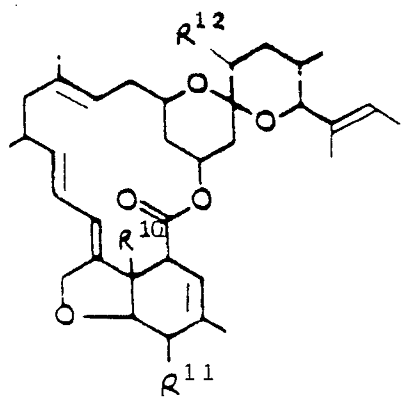

(VIII)

wherein $R^{10}$ is hydroxy or methoxy and $R^{11}$ and $R^{12}$ are the same or different and each is selected from optionally protected hydroxy, alkoxy, acyloxy, sulphonyloxy, oxo and optionally O-substituted oximino, but excluding VM44864 and VM44866 as hereinbefore defined.

2. A compound according to claim 1, wherein (a) $R^{11}$ is alkoxy, $R^{12}$ is hydroxy or alkoxy, and $R^{10}$ is hydroxy or methoxy, with the proviso that when $R^{10}$ is methoxy $R^{12}$ is also methoxy, or (b) $R^{10}$ is hydroxy, $R^{11}$ is methoxy, acyloxy or sulphonyloxy and $R^{12}$ is acyloxy or sulphonyloxy, or (c) $R^{10}$ is hydroxy, $R^{11}$ is methoxy or oxo and $R^{12}$ is oxo.

3. A compound selected from the group consisting of: 5-keto VM44866, 22-methoxy VM44864, 7,22-dimethoxy VM44864, 22-methanesulphonyloxy VM44864, 22-acetoxy VM44864, 22-Keto VM44864, 22-Keto VM44866, 22-hydroxyimino VM44864, 5-hydroxyimino VM44866, 22-methoxyimino VM44864, 22-allyloxyimino VM44864, the 22 epimer of VM44864, the 22 epimer of 22-acetoxy VM44864, the 22 epimer of VM44866, and 22-hydroxyimino VM44866, wherein VM44864 and VM44866 are as hereinbefore defined.

4. A process for the preparation of a compound of formula (VIIIA):

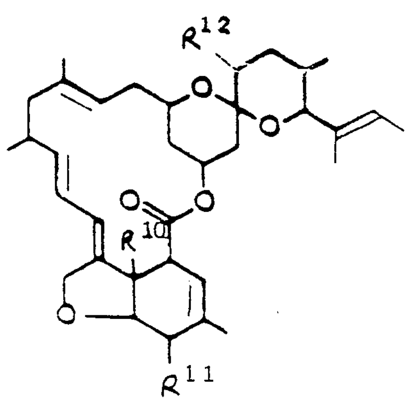

(VIIIA)

wherein $R^{10}$ is hydroxy or methoxy and $R^{11}$ and $R^{12}$ are the same or different and each is selected from optionally protected hydroxy, alkoxy, acyloxy, sulphonyloxy, oxo and optionally O-substituted oximino which process comprises subjecting a different compound of formula (VIIIA) to any one or more of the following steps:

(a) protection, deprotection, alkylation, acylation, sulphonylation, or oxidation of a hydroxy group,
(b) conversion of a 5-methoxy group to a 5-oxo group,
(c) reduction of an oxo group, and
(d) conversion of an oxo to an optionally O-substituted oxyimino group.

5. A process according to claim 4, wherein the said different compound of formula (VIIIA) is VM44864 or VM44866 as hereinbefore defined.

6. A process according to claim 4, which comprises (a) methylating VM44866 to give VM44864, or (b) reducing 5-keto VM44866 to give VM44866, wherein VM44864 and VM44866 are as hereinbefore defined.

7. A compound according to any one of claims 1 to 3, for use in the treatment or prophylaxis of endo- and ectoparasitic infestations of the human or animal body.

8. A method of eradicating arthropod or nematode infestations, which method comprises applying a compound according to any one of claims 1 to 3 or a derivative thereof to the arthropods or nematodes or to their environment.

9. A pesticidal composition comprising a compound according to any one of claims 1 to 3 or a derivative thereof together with an inert carrier or excipient.

10. A pharmaceutical or veterinary composition comprising a compound according to any one of claims 1 to 3 or a pharmaceutically or veterinarily acceptable derivative thereof together with a pharmaceutically or veterinarily acceptable carrier or excipient.